# EUROPEAN PATENT APPLICATION

(11) **EP 2 821 012 A1**
(43) Date of publication of application: **07.01.2015**
(21) Application number: 13755432.5
(22) Date of filing: 28.02.2013
(51) Int. Cl.: A61B 8/00

(54) **ULTRASOUND DIAGNOSTIC EQUIPMENT, MEDICAL DIAGNOSTIC IMAGING EQUIPMENT, AND ULTRASOUND DIAGNOSTIC EQUIPMENT CONTROL PROGRAM**

(30) Priority: 29.02.2012 US 201213408217
(71) Applicant: Kabushiki Kaisha Toshiba, Minato-ku Tokyo 105-8001 (JP); Toshiba Medical Systems Corporation, Otawara-shi, Tochigi 324-8550 (JP)
(72) Inventor: BANJANIN, Zoran, Vernon Hills, Illinois 60061 (US); WOODS, Raymond F., Vernon Hills, Illinois 60061 (US)
(74) Representative: Granleese, Rhian Jane
(86) International application number: PCT/JP2013/055460
(87) International publication number: WO 2013/129590

(57) **Abstract**

According to one embodiment, the an ultrasound diagnosis apparatus acquires an ultrasound data by an ultrasound scanning of a patient via an ultrasound probe, generates an ultrasound image using the ultrasound data and displays the ultrasound image; and is provided with a detection unit, a command generation unit, and a control unit. The detection unit detects a non-touch input instruction from an operator. The command generation unit generates an input command in response to the non-touch input instruction. The control unit controls the ultrasound diagnosis apparatus in response to the input command.

## Description

### FIELD

Embodiments described herein relate generally to ultrasound diagnostic imaging systems for and method of providing a gesture-based user interface for ultrasound diagnostic imaging systems.

### BACKGROUND

In the field of ultrasound medical examination, there have been some attempts to improve a user interface between the ultrasound diagnosis apparatus and the operator. In general, an operator of an ultrasound scanner holds a probe in one hand so that the probe is placed on a patient in an area of interest for scanning an image. The operator observes the image on a display to ascertain accuracy and quality of the image during examination. At the same time, he or she has to adjust imaging parameters from time to time by reaching a control panel using the other hand in order to maintain accuracy and quality of the image.

Despite the above challenging tasks, prior art ultrasound diagnosis apparatus do not provide an easy-to-use interface to the operator. Because the display and the control panel are generally a part of a relatively large scanning device, the image scanning device cannot be placed between the patient and the operator. By the same token, because the operator must reach the control panel, the control panel cannot be placed across the patient from the operator either. For these reasons, the control panel and the display are usually located on the side of the operator within his or her reach.

Consequently, during the use of ultrasound diagnosis apparatus, the operator must extend one hand to the side in order to control knobs (tabs) and switches on the control panel and must hold the probe with the other hand while the operator has to turn his or her head in order to observe the image during the examination. Because of the above described physical requirements, the ultrasound imaging technicians are often subject to occupational injuries over the course of prolong and repetitive operations. Because of the above described physical requirements, the ultrasound imaging technicians are often subject to physical fatigue while operating the apparatus.

As one prior-art attempt provided a hand-held remote control unit instead of the control panel for improving the ultrasound diagnosis apparatus interface. Although the remote control unit alleviated some difficulties, the operator was required to hold the additional piece of equipment in addition to a probe. In other words, the operator's both hands were constantly occupied during the ultrasound imaging session. To adjust any setting that is not accessed through the remote control, the operator had to put the remote control down and later pick it up to resume during scanning. Consequently, the remote control often prevented the operator from easily performing other necessary tasks that require at least one hand during the examination.

As another prior-art attempt provided a voice control unit instead of the control panel for improving the ultrasound diagnosis apparatus interface. Although the voice commands freed the operator from holding any additional piece of equipment other than a probe, the voice command interface experienced difficulties under certain circumstances. For example, since an examination room was not always sufficiently quiet, environment noise prevented the voice control unit from correctly interpreting the voice commands. Another example of the difficulties is accuracy in interpreting the voice command due to various factors such as accents. Although the accuracy might be improved with training to a certain extent, the system needed initial investment and the improvement was generally limited.

In view of the above described exemplary prior-art attempts, the ultrasound diagnosis apparatus still needs an improved operational interface for an operator to control the imaging parameters as well as the operation during the examination sessions.

### BRIEF DESCRIPTION OF THE DRAWINGS

FIG. 1 is a schematic diagram illustrating an ultrasound diagnosis apparatus according to the current embodiment.
FIG. 2A is a diagram illustrating the non-touch input device in the ultrasound diagnosis apparatus according to the current embodiment.
FIG. 2B is a diagram illustrating the non-touch input device for projecting a virtual control panel for inputting commands in the ultrasound diagnosis apparatus according to the current embodiment.
FIG. 3A is a diagram illustrating a first embodiment of the non-touch input device mounted on a top of a display unit in the ultrasound diagnosis apparatus according to the current embodiment.
FIG. 3B is a diagram illustrating second embodiment of the non-touch input device, which is integrated in a top portion of a display unit in the ultrasound diagnosis apparatus according to the current embodiment.
FIG. 3C is a diagram illustrating a third embodiment of the non-touch input device, which is a separate unit that is placed next to a display unit in the ultrasound diagnosis apparatus according to the current embodiment.
FIG. 4 is a diagram illustrating an exemplary operating environment of the ultrasound diagnosis apparatus according to the current embodiment.
FIG. 5 is a diagram illustrating various combinations of the non-touch inputs to the non-touch input device according to the current embodiment.
FIG. 6 is a diagram illustrating another exemplary operating environment of the ultrasound diagnosis apparatus according to the current embodiment.
FIG. 7 is a flow chart illustrating steps or acts involved in one method of processing input commands according to the current embodiment.
FIG. 8 is a flow chart illustrating steps or acts involved in one method of processing gesture commands according to the current embodiment.

### DETAILED DESCRIPTION

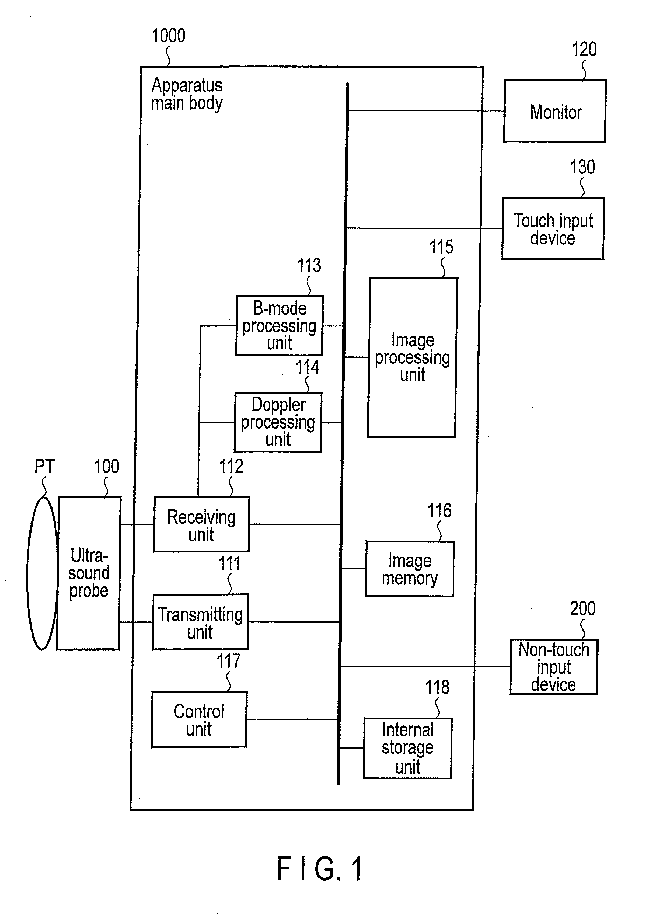

Exemplary embodiments of an ultrasound diagnosis apparatus will be explained below in detail with reference to the accompanying drawings. Now referring to FIG. 1, a schematic diagram illustrates a first embodiment of the ultrasound diagnosis apparatus according to the current embodiment. The first embodiment includes an ultrasound probe 100, a monitor 120, a touch input device 130, a non-touch input device 200 and an apparatus main body 1000. One embodiment of the ultrasound probe 100 includes a plurality of piezoelectric vibrators, and the piezoelectric vibrators generate ultrasound based on a driving signal supplied from a transmitting unit 111 housed in the apparatus main body 1000. The ultrasound probe 100 also receives a reflected wave from a subject Pt and converts it into an electric signal. Moreover, the ultrasound probe 100 includes a matching layer provided to the piezoelectric vibrators and a backing material that prevents propagation of ultrasound backward from the piezoelectric vibrators.

As ultrasound is transmitted from the ultrasound probe 100 to the subject Pt, the transmitted ultrasound is consecutively reflected by discontinuity planes of acoustic impedance in internal body tissue of the subject Pt and is also received as a reflected wave signal by the piezoelectric vibrators of the ultrasound probe 100. The amplitude of the received reflected wave signal depends on a difference in the acoustic impedance of the discontinuity planes that reflect the ultrasound. For example, when a transmitted ultrasound pulse is reflected by a moving blood flow or a surface of a heart wall, a reflected wave signal is affected by a frequency deviation. That is, due to the Doppler effect, the reflected wave signal is dependent on a velocity component in the ultrasound transmitting direction of a moving object.

The apparatus main body 1000 ultimately generates signals representing an ultrasound image. The apparatus main body 1000 controls the transmission of ultrasound from the probe 100 towards a region of interest in a patient as well as the reception of a reflected wave at the ultrasound probe 100. The apparatus main body 1000 includes a transmitting unit 111, a receiving unit 112, a B-mode processing unit 113, a Doppler processing unit 114, an image processing unit 115, an image memory 116, a control unit 117 and an internal storage unit 118, all of which are connected via internal bus.

The transmitting unit 111 includes a trigger generating circuit, a delay circuit, a pulsar circuit and the like and supplies a driving signal to the ultrasound probe 100. The pulsar circuit repeatedly generates a rate pulse for forming transmission ultrasound at a certain rate frequency. The delay circuit controls a delay time in a rate pulse from the pulsar circuit for utilizing each of the piezoelectric vibrators so as to converge ultrasound from the ultrasound probe 100 into a beam and to determine transmission directivity. The trigger generating circuit applies a driving signal (driving pulse) to the ultrasound probe 100 based on the rate pulse.

The receiving unit 112 includes an amplifier circuit, an analog-to-digital (A/D) converter, an adder and the like and creates reflected wave data by performing various processing on a reflected wave signal that has been received at the ultrasound probe 100. The amplifier circuit performs gain correction by amplifying the reflected wave signal. The A/D converter converts the gain-corrected reflected wave signal from the analog format to the digital format and provides a delay time that is required for determining reception directivity. The adder creates reflected wave data by adding the digitally converted reflected wave signals from the A/D converter. Through the addition processing, the adder emphasizes a reflection component from a direction in accordance with the reception directivity of the reflected wave signal. In the above described manner, the transmitting unit 111 and the receiving unit 112 respectively control transmission directivity during ultrasound transmission and reception directivity during ultrasound reception.

The apparatus main body 1000 further includes the B-mode processing unit 113 and the Doppler processing unit 114. The B-mode processing unit 113 receives the reflected wave data from the receiving unit 112, performs logarithmic amplification and envelopes detection processing and the like so as to create data (B-mode data) that a signal strength is expressed by the brightness. The Doppler processing unit 114 performs frequency analysis on velocity information from the reflected wave data that has been received from the receiving unit 112. The Doppler processing unit 114 extracts components of a blood flow, tissue, and contrast media echo by Doppler effects. The Doppler processing unit 114 generates Doppler data on moving object information such as an average velocity, a distribution, power and the like with respect to multiple points.

The apparatus main body 1000 further includes additional units that are related to image processing of the ultrasound image data. The image processing unit 115 generates an ultrasound image from the B-mode data from the B-mode processing unit 113 or the Doppler data from the Doppler processing unit 114. Specifically, the image processing unit 115 respectively generates a B-mode image from the B-mode data and a Doppler image from the Doppler data. Moreover, the image processing unit 115 converts or scan-converts a scanning-line signal sequence of an ultrasound scan into a predetermined video format such as a television format. The image processing unit 115 ultimately generates an ultrasound display image such as a B-mode image or a Doppler image for a display device. The image memory 116 stores ultrasound image data generated by the image processing unit 115.

The control unit 117 controls overall processes in the ultrasound diagnosis apparatus. Specifically, the control unit 117 controls processing in the transmitting unit 111, the receiving unit 112, the B-mode processing unit 113, the Doppler processing unit 114 and the image processing unit 115 based on various setting requests that are inputted by the operator via the input devices, control programs and setting information that are read from the internal storage unit 118. For Example, the control programs executes certain programmed sequence of instructions for transmitting and receiving ultrasound, processing image data and displaying the image data. The setting information includes diagnosis information such as a patient ID and a doctor's opinion, a diagnosis protocol and other information. Moreover, the internal storage unit 118 is optionally used for storing images stored in the image memory 116. Certain data stored in the internal storage unit 118 is optionally transferred to an external peripheral device via an interface circuit. Lastly, the control unit 117 also controls the monitor 120 for displaying an ultrasound image that has been stored in the image memory 116.

A plurality of input devices exists in the first embodiment of the ultrasound diagnosis apparatus according to the current embodiment. Although the monitor or display unit 120 generally displays an ultrasound image as described above, a certain embodiment of the display unit 120 additionally functions as an input device such as a touch panel alone or in combination with other input devices for a system user interface for the first embodiment of the ultrasound diagnosis apparatus. The display unit 120 provides a Graphical User Interface (GUI) for an operator of the ultrasound diagnosis apparatus to input various setting requests in combination with the input device 130. The input device 130 includes a mouse, a keyboard, a button, a panel switch, a touch command screen, a foot switch, a trackball, and the like. A combination of the display unit 120 and the input device 130 optionally receives predetermined setting requests and operational commands from an operator of the ultrasound diagnosis apparatus. The combination of the display unit 120 and the input device 130 in turn generates a signal or instruction for each of the received setting requests and or commands to be sent to the apparatus main body 1000. For example, a request is made using a mouse and the monitor to set a region of interest during an upcoming scanning session. Another example is that the operator specifies via a processing execution switch a start and an end of image processing to be performed on the image by the image processing unit 115.

The above described input modes generally require an operator to touch a certain device such as a switch or a touch panel to generate an input signal. Since any of the touch input modes requires an operator to reach a corresponding input device at least with one hand while the operator is holding a probe with the other hand, it has been challenging under certain circumstances during a scanning session.

Still referring to FIG. 1, a plurality of input devices in the first embodiment of the ultrasound diagnosis apparatus according to the current embodiment additionally includes a non-touch input device 200. One embodiment of the non-touch input device 200 is connected to the apparatus main body 1000 via predetermined wired or wireless connection for receiving non-contact inputs such as commands and data for operating the ultrasound diagnosis apparatus according to the current embodiment. For example, the non-contact input includes at least a predetermined set of gestures, and the non-touch input device 200 receives at least a predetermined gesture. The gesture command is optionally a predetermined hand gesture that is either stationary or moving with respect to the non-touch input device 200. However, the gesture is not limited to a hand gesture and optionally includes any non-contacting body posture and or movement. One example of the body movement is nodding that is optionally included as a predetermined gesture to be recognized by the non-touch input device 200.

The non-touch input device 200 additionally recognizes non-contact inputs that are not necessarily based upon gestures or body posture of the user. The non-contact input to be recognized by the non-touch input device 200 optionally includes a relative position and a type of the ultrasound probe 100. For example, when the probe 100 is moved off from a patient, the non-touch input device 200 generates an input signal to the apparatus main body 1000 to freeze a currently available image. By the same token, when the non-touch input device 200 detects a probe 100, the non-touch input device 200 generates an input signal to the apparatus main body 1000 for setting certain predetermined scanning parameters that are desirable for the detected type of the probe 100. Furthermore, the non-contact inputs to be recognized by the non-touch input device 200 optionally include audio or voice commands. For the above reasons, the non-touch input device 200 is synonymously called a hand-free user interface device in the sense that a user does not reach and touch a predetermined input device.

In the first embodiment of the ultrasound diagnosis apparatus according to the current embodiment, the non-touch input device 200 is not necessarily limited to perform the above described functions in an exclusive manner. In other embodiments of the ultrasound diagnosis apparatus according to the current embodiment, the non-touch input device 200 performs together with other devices such as the image processing unit 115 and the control unit 117 to accomplish the above described functions.

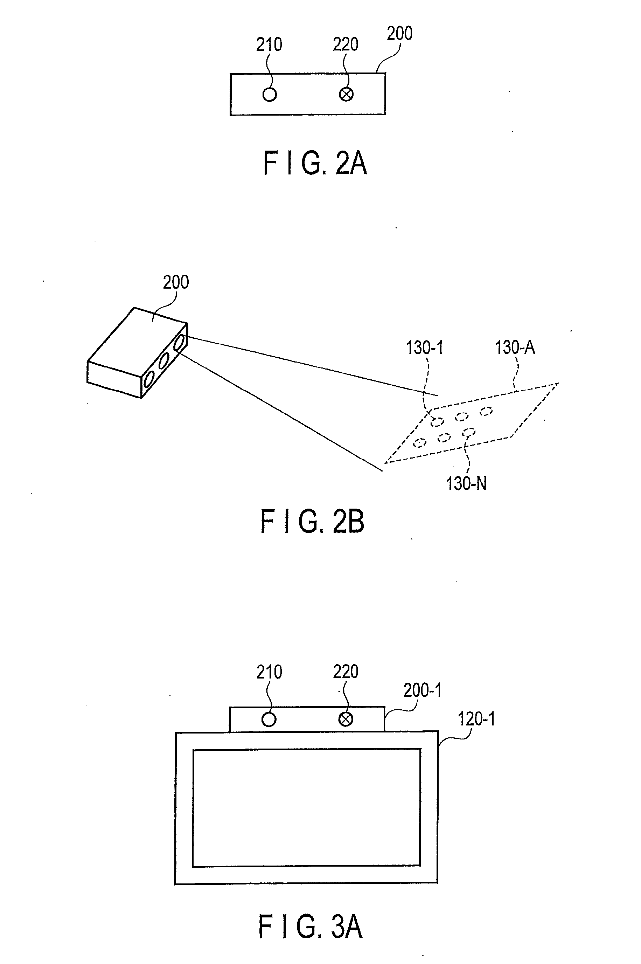

Now referring to FIG. 2A, a diagram illustrates one embodiment of the non-touch input device 200 in the ultrasound diagnosis apparatus according to the current embodiment. The non-touch input device 200 generally includes an infra red (IR) light source and certain sensors such as an IR light sensor. The non-touch input device 200 optionally includes any combination of an image optical sensor, a 3D camera, an ultrasound transmitter, an ultrasound receiver and an accelerometer. The above sensors in the non-touch input device 200 alone or in combination with other sensors detect a shape, a depth and or a movement of a person so as to determine if a predetermined gesture is input. The above sensors are merely illustrative, and the non-touch input device 200 according to the current embodiment is not limited to a particular set of sensors or sensing modes for detecting a gesture command or a non-contacting hand-free signal from a person, an operator or a user. For example, other sensing elements include an ultrasound transmitter and an ultrasound receiver for detecting a gesture command or a non-contacting hand-free signal from a user. To facilitate the detection, the user optionally may wear a lively colored glove so that a hand gesture is visibly enhanced. One exemplary embodiment of the non-touch input device 200 includes an IR light 210 and a depth image detector 220 for detecting a predetermined gesture so as to generate a corresponding input command according to the current embodiment.

Furthermore, relating each gesture of the operator to each input command for controlling the ultrasound diagnosis apparatus can be optionally set and registered, in addition to the initial corresponding relations set in the apparatus. For example, the registration is performed by inputting the corresponding relations via a touch input device 130 or a non-touch input device 200, or by obtaining the preset corresponding relations data via network. Such a table of corresponding relations between each gesture and each input command is stored and managed in a store unit not shown in FIG. 1.

Still referring to FIG. 2A, one embodiment of the non-touch input device 200 optionally includes at least one microphone for sensing a voice command from a user in addition to the above described gesture command detectors. The non-touch input device 200 optionally detects voice commands in combination with the above described gesture commands. The voice commands are supplemental to the gesture commands under certain circumstances while the voice commands are alternative to the gesture commands under other circumstances. For example, after the user inputs a gesture commands such as "change scan depth," a parameter is needed as to which depth. Although the user optionally gestures a predetermined additional hand gesture for a particular depth as a parameter to the "change scan depth" command, the user instead inputs a voice command for a desirable depth following the "change scan depth" gesture command if the operating environment is sufficiently quiet. Repeated changes in scan depth may be easily accomplished by the voice commands as supplement to the initial change depth gesture command.

With respect to a voice command, one embodiment of the non-touch input device 200 optionally includes a microphone and an associated circuit for selectively processing the voice commands. For example, one embodiment of the non-touch input device 200 selectively filters the voice command for a predetermined person. In another example, the non-touch input device 200 selectively minimizes certain noise in the voice commands. Noise cancelation is achieved by use of multiple microphones and space selective filtering of room and system noises. Furthermore, the non-touch input device 200 optionally associates certain voice commands with selected gesture commands and vice versa. The above described additional functions of the non-touch input device 200 require predetermined parameters that are generally input during a voice command training session prior to the examination.

Referring to FIG. 2B, a diagram illustrates one embodiment of the non-touch input device 200 for projecting a virtual control panel 130-A in the ultrasound diagnosis apparatus according to the current embodiment. In one embodiment, the non-touch input device 200 includes a hologram projector for projecting the virtual control panel 130-A in the vicinity of the user. One implementation of the virtual control panel 130-A closely resembles the touch input device 130 in appearance and includes virtual switches and knobs 130-1 through 130-N that correspond to the hand-control mechanisms of the touch input device 130. One embodiment of the non-touch input device 200 continuously detects the user hand position with respect to the projected virtual control panel 130-A and a certain predetermined hand movement for controlling any of the virtual switches and knobs 130-1 through 130-N as indicated by dotted lines. Upon detecting the predetermined hand movement such as turning a knob or flipping a switch within a relative distance from one of the projected image portions 130-1 through 130-N, the non-touch input device 200 generates a corresponding input command according to the current embodiment. According to the current embodiment, the projected virtual control panel 130-A is not limited to a particular set of the control switches and or knobs of the real control panel of the touch input device 130.

In the embodiment of the ultrasound diagnosis apparatus according to the current embodiment, the non-touch input device 200 is not necessarily limited to perform the above described functions in an exclusive manner. In other embodiments of the ultrasound diagnosis apparatus according to the current embodiment, the non-touch input device 200 performs together with other devices such as the image processing unit 115 and the control unit 117 to accomplish the above described functions.

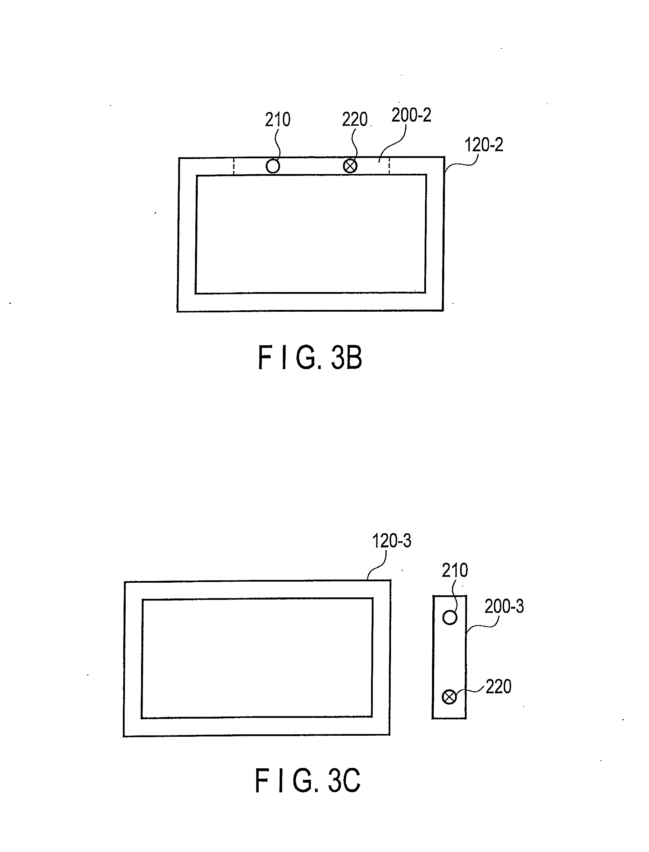

Now referring to FIGS. 3A, 3B and 3C, the non-touch input device 200 is implemented in various manners in the ultrasound diagnosis apparatus according to the current embodiment. FIG. 3A illustrates a first embodiment of a non-touch input device 200-1, which is mounted on a top of a display unit 120-1. The mounting is not limited on the top of the display unit 120-1 and includes any other surfaces of the display unit 120-1 or even other units or devices in the ultrasound diagnosis apparatus according to the current embodiment. Depending upon implementation, the non-touch input device 200-1 is optionally mounted on the display unit 120-1 in a retrofitted manner in an existing ultrasound diagnosis apparatus system. One embodiment of the non-touch input device 200-1 includes the IR light 210 and the depth image detector 220 according to the current embodiment.

FIG. 3B illustrates a second embodiment of a non-touch input device 200-2, which is integrated in a top portion of a display unit 120-2 as indicated by the dotted lines. The integration is not limited to the top portion of the display unit 120-2 and includes any other portions of the display unit 120-2 or even other units or devices in the ultrasound diagnosis apparatus according to the current embodiment. One embodiment of the non-touch input device 200-2 includes the IR light 210 and the depth image detector 220 according to the current embodiment.

FIG. 3C illustrates a third embodiment of a non-touch input device 200-3, which is a separate unit that is placed next to a display unit 120-3. The placement is not limited to the side of the display unit 120-3 and includes any other locations of the display unit 120-3 or even other units or devices in the ultrasound diagnosis apparatus according to the current embodiment. Depending upon implementation, the non-touch input device 200-3 is optionally placed near the display unit 120-3 or other devices in a retrofitted manner in an existing ultrasound diagnosis apparatus system. One embodiment of the non-touch input device 200-3 includes the IR light 210 and the depth image detector 220 according to the current embodiment.

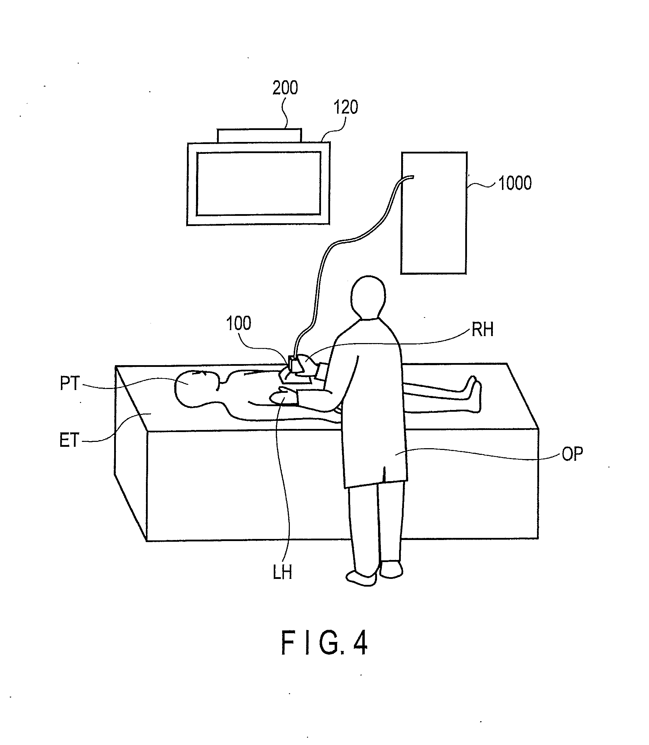

Now referring to FIG. 4, a diagram illustrates an exemplary operating environment of one embodiment of the ultrasound diagnosis apparatus according to the current embodiment. In an exemplary environment, a patient PT is laid on an examination table ET while an operator OP holds the probe 100 with one hand and places it on the patient PT for scanning an ultrasound image. The probe 100 is wired or wirelessly connected to the main body 1000, which is placed on the other side of the patient PT from the operator OP. The operator usually stands in front of the examination table ET and directly faces the patient PT and the display unit 120, which is also placed across the patient PT for easy viewing. In the exemplary embodiment, the non-touch input device 200 is mounted on top of the display unit 120 and moves together with the display unit 120 as the display unit 120 is adjustably positioned for the operator OP.

Still referring to FIG. 4, in the same exemplary environment, as the operator OP holds the probe 100 with his or her right hand RH and scans it over the patient PT, the operator OP directly faces the display unit 120. In this forward-looking posture, the operator OP inputs predetermined gesture commands with his or her left hand LH to the non-touch input device 200 according to one exemplary operation of the current embodiment. In turn, the non-touch input device 200 receives the gesture commands and generates corresponding input signals to the main body 1000 for performing the operations as specified by the gesture commands. Accordingly, in the illustrated operating environment, the operator OP is substantially free from turning his or her body and reaching the knobs and the switches on a prior art control panel that is generally located at a lateral side of the operator OP. In other words, the operator OP maintains a substantially forward-looking posture for monitoring the display unit 120 and inputting the commands during the scanning session. Furthermore, since no equipment is located between the operator OP and the examination table ET, the operator is substantially unobstructed to move around the examination table ET during the scanning session.

In the above described exemplary environment, one embodiment of the non-touch input device 200 processes various types of inputs while the operator OP examines the patient using the ultrasound diagnosis apparatus according to the current embodiment. The input commands are not necessarily related to the direct operation of the ultrasound imaging diagnosis apparatus and include optional command for annotations, measurements and calculations in relation to the ultrasound images that have been acquired during the examination session. For example, the annotations include information on the region of interest, the patient information, the scanning parameters and the scanning conditions. An exemplary measurement includes a size of a certain tissue area such as a malignant tumor in the ultrasound images that have been acquired during the examination session. An exemplary calculation result in certain values such as a heart rate and blood flow velocity based upon the acquired ultrasound data that have been acquired during the examination session.

The embodiment of FIG. 4 is merely illustrative and is limited to the above described particular features of the exemplary embodiment in order to practice the current embodiment. For example, any combination of wired or wireless connections is applicable among the probe 100, the display unit 120, the non-touch input device 200 and the main body 1000 in practicing the current embodiment. Also in practicing the current embodiment, the location of the display unit 120 and the non-touch input device 200 is not limited to be directly in front of the operator OP and across the patient PT. By the same token, the operator is not required to hold the probe 100 with any particular hand in practicing the current embodiment and optionally switches hands or uses both hands to hold the probe 100 during the examination session. Furthermore, the above described exemplary embodiment is optionally combined with the input device 130 for receiving predetermined setting requests and operational commands from an operator of the ultrasound diagnosis apparatus. The input device 130 includes a mouse, a keyboard, a button, a panel switch, a touch command screen, a foot switch, a trackball, and the like. Lastly, the non-touch input device 200 optionally receives audio or voice commands.

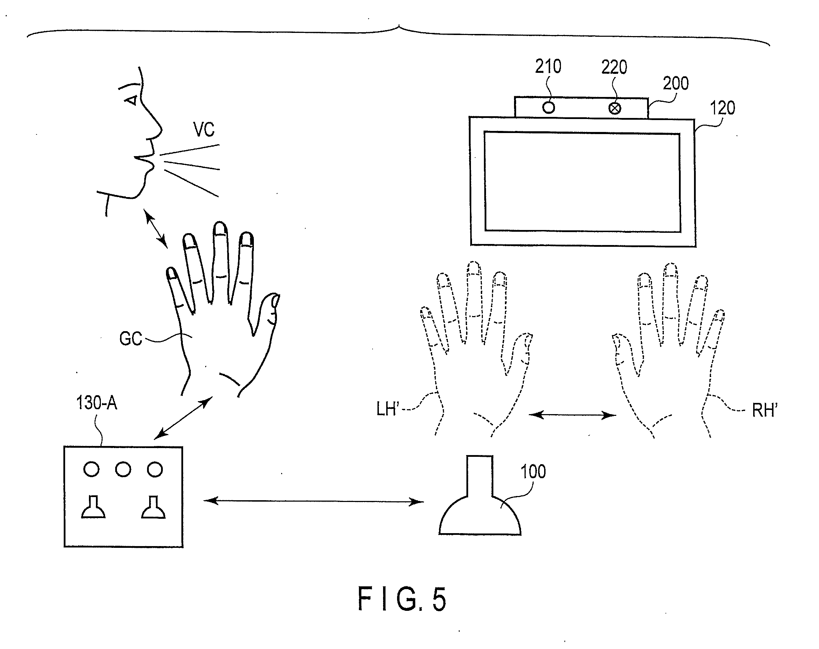

FIG. 5 is a diagram illustrating various inputs to the non-touch input device 200 according to the current embodiment. One embodiment of the non-touch input device 200 is mounted on the display monitor 120 and includes at least a pair of the IR light 210 and the depth image detector 220. For example, the non-touch input device 200 detects a predetermined gesture command GC as articulated by the operator so as to generate a corresponding input signal to the main body 1000 for performing the corresponding operation. In another example, the non-touch input device 200 also detects a predetermined voice command VC as articulated by the same operator. The non-touch input device 200 optionally includes a voice command unit and or a virtual control panel unit that are not shown in the diagram. In an alternative embodiment of the non-touch input device 200, a voice command unit and or a virtual control panel unit are provided as separate units from the non-touch input device 200.

Still referring to FIG. 5, the non-touch input device 200 allows the operator to change the input mode as well as the input source in a flexible manner. For example, during a sequence of predetermined gestures, the operator is allowed to switch hands between a left hand LH' and a right RH' as indicated by a double-headed arrow. By the same token, the non-touch input device 200 receives from the operator a combination of the predetermined gesture command GC and the predetermined voice command VC as indicated by a vertical double-headed arrow. The input mode such as voice and gesture is optionally changed even during a single operational command. Furthermore, the non-touch input device 200 automatically generates a certain input signal to the main body 1000 without a voice or gesture command from the user. Since a certain embodiment of the non-touch input device 200 continuously detects a relative position of the probe 100 with respect to a patient, when the probe 100 is no longer on the patient body surface, the non-touch input device 200 generates the input signal corresponding to a "freeze an image" command so that the last available image is maintained on the monitor 120. In the above example, the input source is optionally changed from the operator to the probe. Furthermore, the use of the virtual control panel 130-A is optionally combined with any other input mode or source. For the use of the virtual control panel 130-A, the detection of the hand position and the hand movement have been described with respect to FIG. 2B.

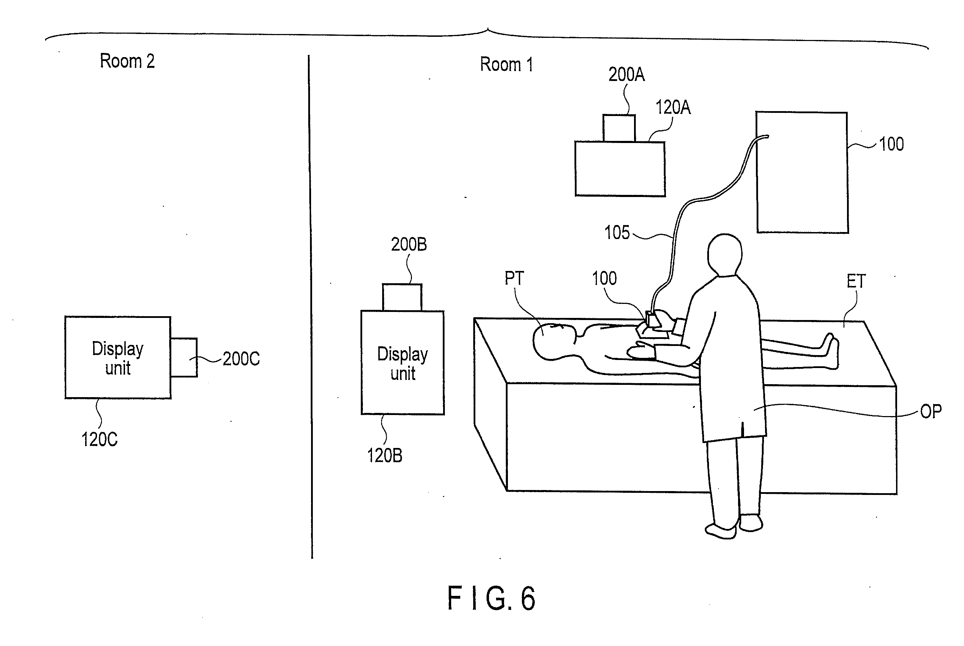

Now referring to FIG. 6, a diagram is merely illustrative and is limited to the above described particular features illustrates an exemplary environment where one embodiment of the ultrasound diagnosis apparatus is operated according to the current embodiment. In an exemplary embodiment, there exist multiple ones of the non-touch input device 200 and multiple sets of the touch input device 130 and the display unit 120 in the ultrasound diagnosis apparatus. In another embodiment, only multiple sets of the display unit 120 exist in the ultrasound diagnosis apparatus. In either embodiment, a combination of the non-touch input device 200 and the display unit 120 is located at a plurality of locations such as different rooms in the same building and or geographically remote locations anywhere in the world.

Still referring to FIG. 6, a diagram illustrates one example where a patient PT is laid on an examination table ET in Room 1. An operator OP holds the probe 100 with one hand and places it on the patient PT for scanning an ultrasound image. The probe 100 is wired or wirelessly connected to the main body 1000, which is placed across the patient PT from the operator OP. The operator usually stands in front of the examination table ET and directly faces the patient PT and the display unit 120A, which is also placed across the patient PT for comfortable viewing. In the exemplary embodiment, the non-touch input device 200A is mounted on top of the display unit 120A and moves together with the display unit 120A as the display unit 120A is adjustably positioned for the operator OP.

In this example, another set of a non-touch input device 200B and a display unit 120B is also located in Room 1 for additional personnel who are not illustrated in the diagram. The additional personnel in Room 1 either passively observe the display unit 120B or actively participate in the scanning session by articulating operational commands to the non-touch input device 200B during the same scanning session as the operator OP scans the patient PT via the probe 100. For example, several students simply observe the scanning session through the display monitor 120B for learning the operation of the ultrasound diagnosis apparatus. Another example is that a doctor articulates an operational command such as a predetermined gesture to the non-touch input device 200B for acquiring additional images that are not recorded by the operator OP as the doctor observes the scanning session via the display monitor 120B. In case of anticipating multiple operational commands from various input devices, a rule is established in advance and stored in the main body 1000 for resolving conflicting commands or prioritizing a plurality of commands.

Still referring to FIG. 6, a diagram also illustrates yet another set of a non-touch input device 200C and a display unit 120C, which is located in Room 2 for additional personnel who are not illustrated in the diagram. In one implementation, Room 2 is located in the same building as Room 1. For example, Room 1 is an operating room while Room 2 is an adjacent observation room. Alternatively, in another implementation, Room 2 is located at a different location from Room 1 outside of the building anywhere in the world. For example, Room 1 is an examination room in one city while Room 2 is a doctor's office in another city. In any case, the additional personnel in Room 2 either passively observe the display unit 120C or actively participate in the scanning session by articulating operational commands to the non-touch input device 200C during the same scanning session as the operator OP scans the patient via the probe 100 in Room 1. For example, several students simply observe the scanning session via the display monitor 120C for learning the operation of the ultrasound diagnosis apparatus. Another example is that a doctor articulates an operational command such as a predetermined gesture to the non-touch input device 200C for acquiring additional images that are not recorded by the operator OP as the doctor observes the scanning session via the display monitor 120C. Yet another example is that a patient is located in an operating room in one city while a doctor in another city observes the operation by remotely using the ultrasound diagnosis apparatus according to the current embodiment so as to offer his or her expert advice for the operation. By providing the multiple input sources for the gesture commands, people a plurality of locations are able to control the scanning images in an interactive manner to share and learn expert knowledge. In case of anticipating multiple operational commands from various input devices, a rule is established in advance and stored in the main body 1000 for resolving conflicting commands or prioritizing a plurality of commands.

The embodiment of FIG. 6 is merely illustrative and is not limited to the above described features of the exemplary embodiment in order to practice the current embodiment. For example, any combination of wired or wireless connections is applicable among the probe 100, the display units 120A-C, the non-touch input devices 200A-C and the main body 1000 in practicing the current embodiment. Also in practicing the current embodiment, the location of the display unit 120A and the non-touch input device 200A is not limited to be directly in front of the operator OP and across the patient PT. By the same token, the operator is not required to hold the probe 100 with any particular hand in practicing the current embodiment and optionally switches hands or uses both hands to hold the probe 100 during the examination session. Furthermore, the above described exemplary embodiment is optionally combined with the input device 130 for receiving predetermined setting requests and operational commands from an operator of the ultrasound diagnosis apparatus. The input device 130 includes a mouse, a keyboard, a button, a panel switch, a touch command screen, a foot switch, a trackball and the like. Lastly, any one of the non-touch input devices 200A-C optionally receives audio or voice commands.

Also, the ultrasound diagnosis apparatus according to the current embodiment is not limited to the above examples. For example, assuming that there are people in one room, such as an operating room etc., the ultrasound diagnosis apparatus is operable to identify a next operator who performs a gesture for input instruction, switch a current operator to the next operator, and receive the non-touch instructions input by the next operator. Moreover, a person who holds the ultrasound probe 100 (i.e. the operator) is identified, and only a gesture performed by the identified operator may be detected as a non-touch instruction. Additionally, the ultrasound diagnosis apparatus according to the current embodiment may identify a person who performed a predetermined action, such as waving a hand etc., and detect only a gesture performed by the identified operator as a non-touch instruction. The ultrasound diagnosis apparatus may re-identify another person who further performed the predetermined action, and detect only a gesture performed by the re-identified operator as a non-touch instruction.

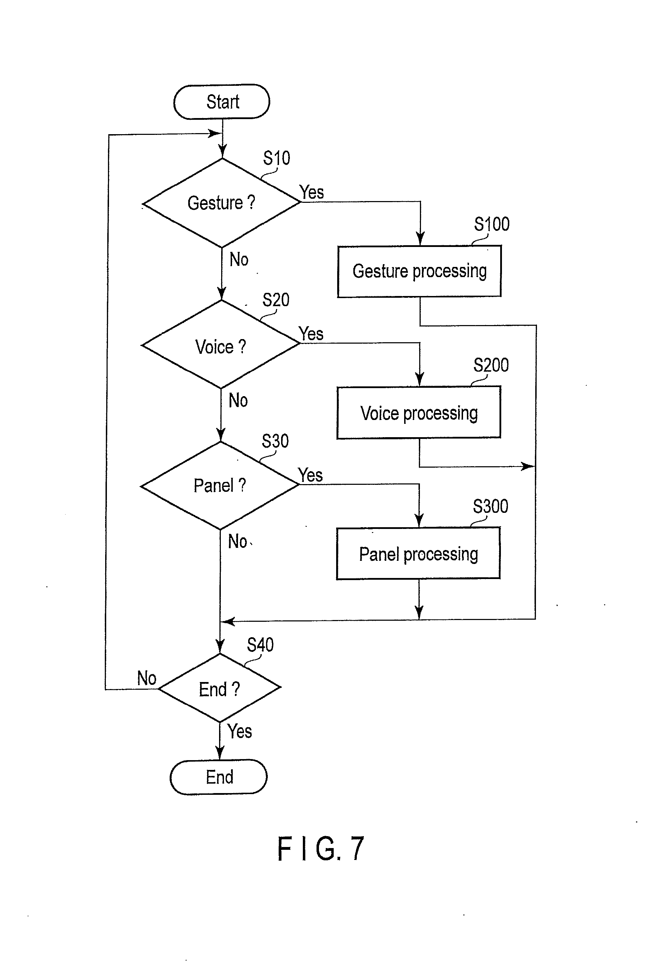

Now referring to FIG. 7, a flow chart illustrates steps or acts involved in one method of processing input commands according to the current embodiment. The exemplary method initially distinguishes and later processes both non-touch input commands as well as touch-input commands. In general, the non-touch input commands include gesture commands and voice commands without touching any physical input devices. On the other hand, the touch input commands involve mechanically or electronically activated signals that are caused by the operator via input devices such as a mouse, a keyboard, a button, a panel switch, a touch command screen, a foot switch, a trackball and the like.

Still referring to FIG. 7, to distinguish a type of an operator input, one exemplary method of the hand-free user interface for an ultrasound diagnosis apparatus performs a series of determining steps as illustrated in the flow chart. In a gesture determining step S10, the exemplary method determines as to whether or not an input is a gesture command. If the input is from a device or unit that processes some movement and or image of the operator, it is determined in the step S10 that the input is a potential gesture command and the exemplary method proceeds to a gesture processing step S100. On the other hand, if the input is not from a device or unit that processes movement and or image of the operator, it is determined in the step S10 that the input is not a potential gesture command and the exemplary method proceeds to a voice determining step S20. If the input is from a device or unit that processes voice of the operator and ambient noise, it is determined in the step S20 that the input is a potential voice command and the exemplary method proceeds to a voice processing step S200. On the other hand, if the input is not from a device or unit that processes voice and or ambient noise, it is determined in the step S20 that the input is not a potential voice command and the exemplary method proceeds to a panel input determining step S30. If the input is from a device or unit that processes tactile inputs from the operator or electrical signals caused by a mechanical input device, it is determined in the step S30 that the input is a potential command from a control panel or other tactile input devices and the exemplary method proceeds to a panel processing step 300. On the other hand, if the input is not from a device or unit that processes tactile inputs, it is determined in the step S30 that the input is not a potential tactile input command and the exemplary method proceeds to an end determining step S40. In the ending step, it is determined as to whether not there is any more input. If there is no input, the exemplary method stops. On the other hand, if there is additional input, the exemplary method goes back to the step S10.

The method of FIG. 7 is merely illustrative and is not limited to the above described steps of the exemplary process in order to practice the current embodiment. Although the exemplary method illustrates the processing steps for gesture, voice and tactile inputs, other processes according to the current embodiment optionally include additional steps of processing other input types such as relative probe positional data. Furthermore, the process is optionally parallel or parallel-serial combination in processing the commands.

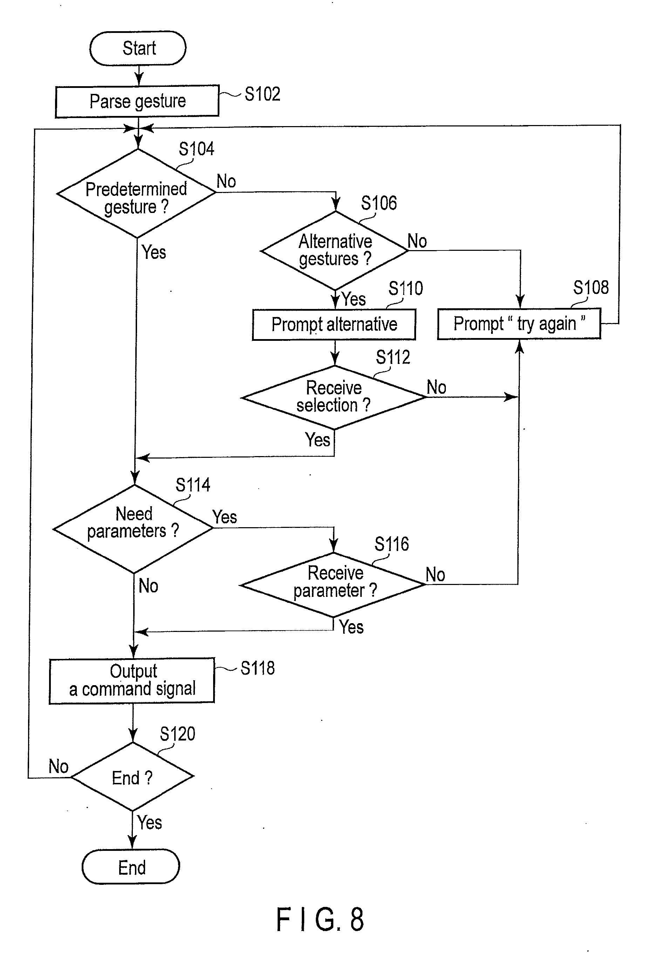

Now referring to FIG. 8, a flow chart illustrates steps or acts involved in one method of processing gesture commands according to the current embodiment. The exemplary flow chart for processing the gesture commands is merely illustrative of one implementation in the hand-free user interface for an ultrasound diagnosis apparatus according to the current embodiment. The gesture processing is not limited to any particular steps in a flow chart and optionally includes additional or alternative steps in order to practice current embodiment. In general, the exemplary gesture processing ultimately determines an output command signal to the ultrasound diagnosis apparatus so that a user specified task is performed according to the gesture command.

Still referring to FIG. 8, a flow chart illustrates steps that ascertain the integrity of the potential gesture command and ultimately generates a corresponding output command signal to the ultrasound diagnosis apparatus according to the current embodiment. In a step S102, the potential gesture command is parsed if the potential gesture command includes more than a single gesture element. After parsing in the step S102, it is generally assumed that a first or initial gesture element is a major command portion in the potential gesture command. Although it is not always the case, the major command portion is often a verb in the potential gesture command. In a step S104, it is determined as to whether or not the first or initial gesture element is one of the predetermined gesture commands. If it is determined in the predetermined gesture determining step S104 that the first or initial gesture element is one of the predetermined gesture commands, the exemplary gesture processing proceeds to a step S114, where it is determined whether or not a parameter is necessary for the first or initial gesture element.

On the other hand, if it is determined in the predetermined gesture determining step S104 that the first or initial gesture element is not one of the predetermined gesture commands, the exemplary gesture processing proceeds to an alternative gesture processing step S106, where an alternative gesture flag has been initialized to a predetermined NO value to indicate a first time. If the alternative gesture flag is NO, the exemplary gesture processing proceeds to prompt in a step S108 a "TRY Again" feedback to the user via audio and or visual prompt on a monitor so that a user can try to repeat the previously unrecognized gesture command or gesture a different gesture command. After the prompt, the alternative gesture processing step S106 sets the alternative gesture flag to a predetermined YES value. Thus, the exemplary gesture processing goes back to the step S104 to process another potential gesture command after the prompting step S108.

In contrast, if the alternative gesture flag is YES in the step S106, the exemplary gesture processing proceeds to a step S110 to prompt a set of the predetermined gesture commands to the user via audio and or visual prompt on a monitor so that the user now selects one of the predetermined gesture commands for the previously unrecognized gesture command or selects a different gesture command. After the prompt in the step S110, the alternative gesture selecting step S112 sets the alternative gesture flag to the predetermined NO value if a selection is received in the step S112 and the exemplary gesture processing proceeds to a step S114. Alternatively, the alternative gesture selecting step S112 leaves the alternative gesture flag to the predetermined YES value if a selection is not received in the step S112 and the exemplary gesture processing goes back to the step S108 and then to the step S104.

After a first or initial gesture element is determined either in the step S104 or S112, the exemplary gesture processing ascertains if a parameter is necessary for the first or initial gesture element in the step S114. If it is determined in the step S114 that a parameter is necessary for the first or initial gesture element according to a predetermined gesture command list, the exemplary gesture processing in a step S116 determines as to whether or not a parameter is received from the user. If the necessary parameter is not received in the step S116, the exemplary gesture processing goes back to the step S108 and then to the step S104. In other words, the exemplary gesture processing requires the user to start the gesture command from the beginning in this implementation. On the other hand, if the necessary parameter is received in the step S116, the exemplary gesture processing proceeds to a step S118, where a corresponding command signal is generated and outputted. If it is determined in the step S114 that a parameter is not necessary for the first or initial gesture element according to the predetermined gesture command list, the exemplary gesture processing also proceeds to the step S118. In an ending step S120, it is determined as to whether not there is any more potential gesture command. If there is no more potential gesture command, the exemplary gesture processing stops. On the other hand, if there are additional potential gesture commands, the exemplary gesture processing goes back to the step S102.

The method of FIG. 8 is merely illustrative and is not limited to the above described steps of the exemplary process in order to practice the current embodiment. Although the exemplary gesture processing requires in this implementation the user to start the gesture command from the beginning in case of failing to match the required parameter in the potential gesture command, other processes according to the current embodiment optionally include additional steps of prompting only for a partial gesture for the required parameter.

Now referring to Table 1, an exemplary set of the gesture commands is illustrated for implementation in the ultrasound diagnosis apparatus according to the current embodiment. Some of the tasks such as "change scan depth" as represented by the gesture commands are related to ultrasound imaging while others such as "select patient data" are generic to other modalities. The left column of Table 1 lists tasks to be performed by the ultrasound diagnosis apparatus according to the current embodiment. The middle column of Table 1 describes prior art user interface for inputting a corresponding command for each of the tasks to be performed by the ultrasound diagnosis apparatus according to the current embodiment. The right column of Table 1 describes a predetermined gesture for each of the tasks to be performed by the ultrasound diagnosis apparatus according to the current embodiment.

**TABLE-US-00001 TABLE 1**

| Task | Conventional U/I | Gesture Command |
|---|---|---|
| Select ultrasound transducer | Press buttons to navigate and select an attached transducer probe. | User picks up the transducer probe and image recognition feature automatically selects that probe. |
| Select patient data/exam type | Press select patient data and exam buttons to navigate and type. | User points at the system display monitor and the hand becomes a pointing device on a virtual control panel. A cursor on the display tracks hand motion. A tapping gesture presses buttons. Open palm gesture to exit virtual control panel. |
| Adjust overall image gain | Turning a knob to increase/decrease overall gain. | Hand gesture mimics twisting motion to adjust overall gain. |
| Change scan depth | Turning a knob to increase/decrease scan depth. | Hand gesture mimics raising and lowering scan depth. |
| Freeze image | Press button to freeze current image. | Remove transducer from patient to freeze on current image. |
| Review acquired images (cine playback after freeze) | Move trackball to review cine images. | Flicking gesture left or right to mimic trackball motion. |
| Zoom in on image | Twist knob to zoom in/out. | Pinching gesture to zoom in/out |

Still referring to Table 1, the list is not limited by the examples in order to practice the current embodiment. The list is merely illustrative and is not a comprehensive list of the commands. The list also illustrates mere exemplary gesture for each of the listed commands, and the same gesture command is optionally implemented by a variety of gestures that the users prefer. In this regard, the gestures are optionally custom-made or selected for each of the gesture command from predetermined gestures. Lastly, as described above with respect to FIG. 6, the gesture commands are used by any combination of the operator and the non-operator of the ultrasound diagnosis apparatus according to the current embodiment.

Moreover, the non-touch input function according to the current embodiment is described an ultrasound diagnosis apparatus. However, it is not necessary to be limited to the exemplifications described in the above embodiment. For example, the non-touch input function according to the current embodiment can be applied to each type of medical image diagnosis apparatus, such as X-ray diagnosis apparatus, X-ray CT imaging apparatus, Magnetic Resonance Imaging apparatus, or Nuclear Medical diagnosis apparatus etc. In addition, the non-touch input function according to the current embodiment also may be applied to a medical image display apparatus, so-called work station, which reads out and displays stored medical images, and performs a measurement and an image processing to observe the medical images.

According to the structure mentioned above, the operator can input a desired command simply and quickly by performing a predetermined gesture without moving or changing the posture to touch an input device. The ultrasound diagnosis apparatus according to the current embodiment can reduce physical fatigue of the operator and also can contribute for performing a quick imaging process. This is especially useful in a case of, for example, an operator of the ultrasound probe and an operator of inputting a desired command to an apparatus are the same person, such as an ultrasound diagnosis apparatus. Conversely, the another operator, who is different from the operator of the probe and the like is operable to input instructions to the ultrasound diagnosis apparatus. This contributes to perform two individual operations of the apparatus and reduce physical fatigue of each operator.

For example, the ultrasound diagnosis apparatus is utilized for monitoring of the diseased part during an operation. In this case, from a hygienic point of view, it is preferable that parts of the ultrasound apparatus, which are in contact with the operator or the patient, should be severely restricted in minimum during the operation. According to the current embodiment, the parts in contact with the operator or the patient can be restricted to only an ultrasound probe because a desired command is able to be inputted by the non-touch input function. As a result, hygiene management can be improved compared to the prior art.

While certain embodiments have been described, these embodiments have been presented by way of example only, and are not intended to limit the scope of the inventions. Indeed, the novel methods and systems described herein may be embodied in a variety of other forms; furthermore, various omissions, substitutions and changes in the form of the methods and systems described herein may be made without departing from the spirit of the inventions. The accompanying claims and their equivalents are intended to cover such forms or modifications as would fall within the scope of the inventions.

Furthermore, the above embodiments are described with respect to examples such as devices, apparatus and methods. Another embodiment to practice the current embodiment of the invention includes computer software such as programs for hand-free operation of the ultrasound system that is loaded into a computer form a recording medium where it is stored.

## Claims

1. An ultrasound diagnosis apparatus which acquires an ultrasound data by an ultrasound scanning of a patient via an ultrasound probe, generates an ultrasound image using the ultrasound data and displays the ultrasound image, the apparatus **characterized by** comprising:
a detection unit configured to detect a non-touch input instruction from an operator;
a command generation unit configured to generate an input command in response to the non-touch input instruction; and
a control unit configured to control the ultrasound diagnosis apparatus in response to the input command.

2. The ultrasound diagnosis apparatus according to claim 1
**characterized by** further comprising a projection unit configured to project an input image at a predetermined position to input a predetermined instruction toward the operator; and
**characterized in that** the detection unit detects the non-touch input instruction input to the projected input image.

3. The ultrasound diagnosis apparatus according to claim 1
**characterized by** further comprising a register unit configured to register a predetermined gesture of the operator corresponding to a predetermined motion of the ultrasound diagnosis apparatus;
**characterized in that** the detection unit detects the predetermined gesture of the operator as the non-touch input; and
the command generation unit generates an input command to exert the predetermined motion of the ultrasound diagnosis apparatus, the predetermined motion being registered by corresponding to the detected non-touch input.

4. The ultrasound diagnosis unit according to claim 1, 2 or 3, **characterized in that**
the detection unit detects a voice input instruction from the operator in addition to the non-touch input instruction; and
the command generation unit generates an input command in response to the voice input instruction.

5. The ultrasound diagnosis apparatus according to claim 1, 2, 3 or 4,
**characterized by** further comprising a determination unit configured to determine the operator; and
**characterized in that** the detection unit detects the non-touch input instruction of the operator determined by the determination unit.

6. The ultrasound diagnosis apparatus according to claim 5, **characterized in that** the determination unit determines the operator based upon a special location of the ultrasound probe.

7. The determination unit according to claim 5, **characterized in that** the determination unit determines the operator who is a detective object of the following non-touch instructions based upon a detection of the predetermined gesture conducted by one of a plurality of operators.

8. The ultrasound diagnosis apparatus according to one of the claims 1 to 7,
**characterized by** further comprising at least one other detection unit which detects a non-touch input instruction from at least one operator who is different from the operator; and
**characterized in that** the command generation unit generates an input command in response to the detected non-touch input instruction by the at least one other detection unit.

9. The ultrasound diagnosis apparatus according to one of the claims 1 to 8,
**characterized in that** the command generation unit generates the input command to freeze an ultrasound image displayed on a display unit in response to the detected non-touch input instructions.

10. The ultrasound diagnosis apparatus according to one of the claims 1 to 9,
**characterized in that** the detection unit detects a type of the ultrasound probe that is connected to the ultrasound diagnosis apparatus body; and
the command generation unit generates the input command which automatically sets at least one parameter for the ultrasound scanning based upon the detected type of the probe.

11. The ultrasound diagnosis apparatus according to one of the claims 1 to 10,
**characterized by** further comprising a touch input detection unit configured to detect an input operation by a touch input; and
**characterized in that** the detection unit detects the non-touch input instruction in response to an input operation is conducted by the touch input detection unit.

12. A medical image diagnosis apparatus which acquires an imaging wave data relating to a patient by imaging the patient, generates a medical image using the imaging wave data, and displays the medical image, the apparatus **characterized by** comprising:
a detection unit configured to detect a non-touch input instruction form an operator;
a command generation unit configured to generate an input command in response to the non-touch input instruction; and
a control unit configured to control the medical image diagnosis apparatus in response to the input command.

13. An ultrasound diagnosis apparatus control program which acquires an ultrasound data by an ultrasound scanning of a patient via an ultrasound probe, generates an ultrasound image using the ultrasound data and displays the ultrasound image, the program **characterized by** comprising:
a detection function that causes a computer to detect a non-touch input instruction form an operator;
a command generation function that causes a computer to generate an input command in response to the non-touch input instruction; and
a control function that causes a computer to control the medical image diagnosis apparatus in response to the input command.

14. A medical image display apparatus which displays a medical image, the apparatus **characterized by** comprising:
a detection unit configured to detect a non-touch input instruction from an operator;
a command generation unit configured to generate an input command in response to the non-touch input instruction; and
a control unit configured to control the medical image display apparatus in response to the input command.
